# EUROPEAN PATENT APPLICATION

(11) **EP 4 327 844 A1**
(43) Date of publication of application: **28.02.2024**
(21) Application number: 22804384.0
(22) Date of filing: 24.03.2022
(51) Int. Cl.: A61M 1/36, B01J 4/00

(54) **LIQUID STORAGE TANK**

(30) Priority: 21.05.2021 JP 2021085971
(71) Applicant: Nipro Corporation, Osaka-shi, Osaka 531-8510 (JP)
(72) Inventor: ISHIHARA, Kazuhisa, Osaka-shi, Osaka 531-8510 (JP); KUWAHARA, Yuji, Osaka-shi, Osaka 531-8510 (JP); KOISO, Eiichi, Osaka-shi, Osaka 531-8510 (JP)
(74) Representative: Prüfer & Partner mbB Patentanwälte · Rechtsanwälte
(86) International application number: PCT/JP2022/013987
(87) International publication number: WO 2022/244466

(57) **Abstract**

A liquid storage reservoir (1) includes a housing (100), an inflow port (200), an outflow port (300), and an introduction pipe (400) connected to a downstream-side end portion of the inflow port (200) to introduce a liquid into the housing (100). The inflow port (200) is connected to the housing (100) in a posture intersecting a vertical direction. The introduction pipe (400) is formed to be tubular, and has a shape extending vertically downward from the downstream-side end portion of the inflow port (200). The housing (100) has an opposed wall (121) formed at a position opposed to a lower end portion of the introduction pipe (400), and a guide wall (122) that guides the liquid downward from the opposed wall (121).

## Description

### TECHNICAL FIELD

The present invention relates to a liquid storage reservoir.

### BACKGROUND ART

A liquid storage reservoir that stores a liquid such as a cardiac muscle protection liquid has been conventionally known. For example, Japanese Patent Laying-Open No. 2015-100609 discloses a cardiac muscle protection liquid reservoir including: a reservoir housing that forms a storage space for a cardiac muscle protection liquid; an inflow port provided at an upper portion of the reservoir housing; and an outflow port provided at a lower portion of the reservoir housing. A stirring sheet is provided in the reservoir housing, and has an introduction end portion to which an introduction pipe communicating with the inflow port is coupled. In order to eject the liquid from the introduction pipe, the diameter of the introduction pipe is gradually decreased in a direction toward its tip.

### CITATION LIST

### PATENT LITERATURE

PTL 1: Japanese Patent Laying-Open No. 2015-100609

### SUMMARY OF INVENTION

### TECHNICAL PROBLEM

In the liquid storage reservoir such as the one described in Japanese Patent Laying-Open No. 2015-100609, it is preferable to reduce bubbles formed in the cardiac muscle protection liquid flowing out of the outflow port.

An object of the present invention is to provide a liquid storage reservoir to suppress formation of bubbles in a liquid flowing out of an outflow port.

### SOLUTION TO PROBLEM

A liquid storage reservoir according to an aspect of the present invention includes: a housing that is able to store a liquid; an inflow port connected to an upper portion of the housing to allow the liquid to flow into the housing; an outflow port connected to a lower portion of the housing to allow the liquid to flow out of the housing; and an introduction pipe connected to a downstream-side end portion of the inflow port to introduce the liquid into the housing, wherein the inflow port is connected to the housing in a posture intersecting a vertical direction, the introduction pipe is formed to be tubular, and has a shape extending vertically downward from the downstream-side end portion of the inflow port, the housing has an opposed wall formed at a position opposed to a lower end portion of the introduction pipe, and a guide wall that guides the liquid downward from the opposed wall.

### ADVANTAGEOUS EFFECTS OF INVENTION

According to the present invention, it is possible to provide a liquid storage reservoir to suppress formation of bubbles in a liquid flowing out of an outflow port.

### BRIEF DESCRIPTION OF DRAWINGS

Fig. 1 is a perspective view of a liquid storage reservoir according to an embodiment of the present invention.
Fig. 2 is a plan view of the liquid storage reservoir shown in Fig. 1.
Fig. 3 is a rear view of the liquid storage reservoir shown in Fig. 1.
Fig. 4 is a cross sectional view along a line IV-IV in Figs. 2 and 3.
Fig. 5 is an enlarged view of a range indicated by a solid line V in Fig. 4.

### DESCRIPTION OF EMBODIMENTS

Embodiments of the present invention will be described with reference to figures. It should be noted that in the below-described figures, the same or corresponding members are denoted by the same reference characters.

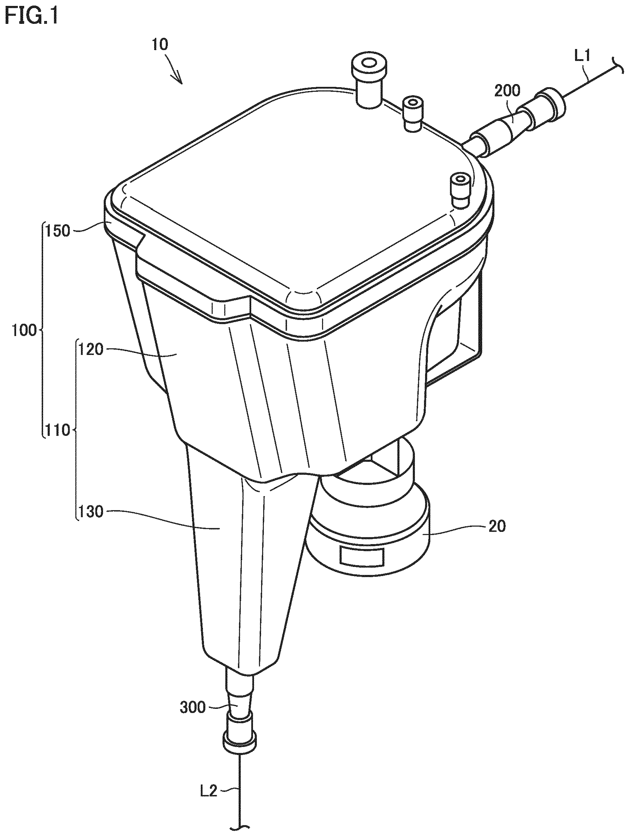

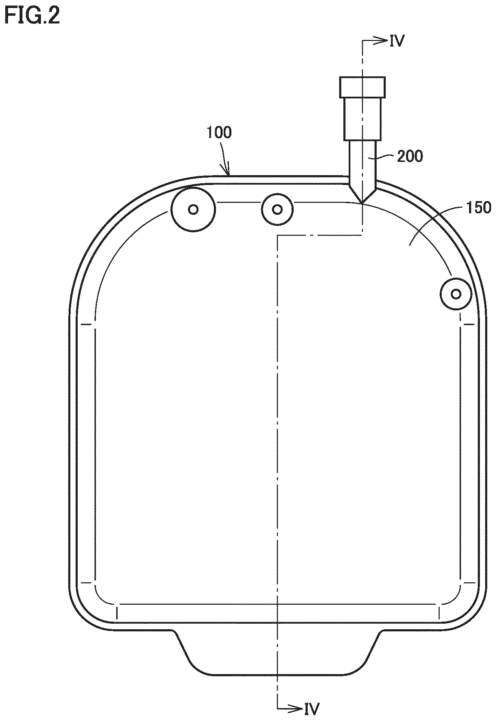

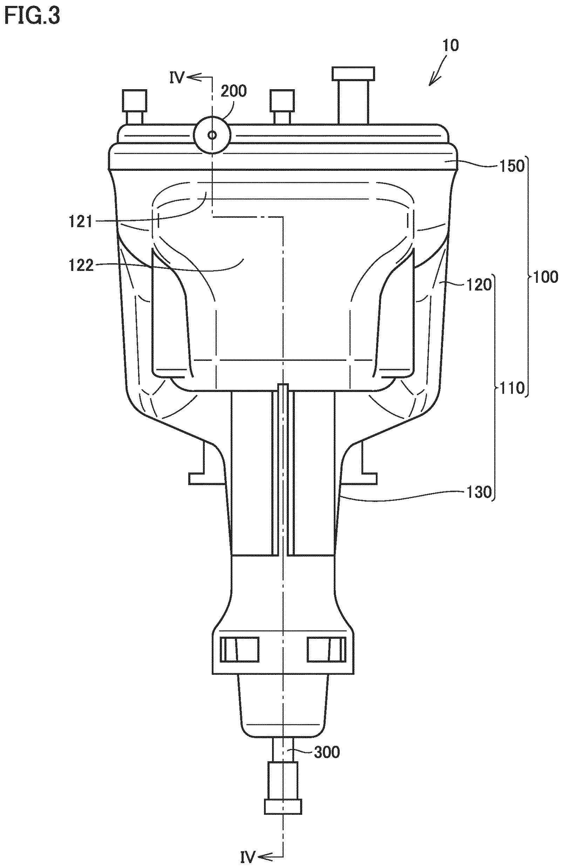

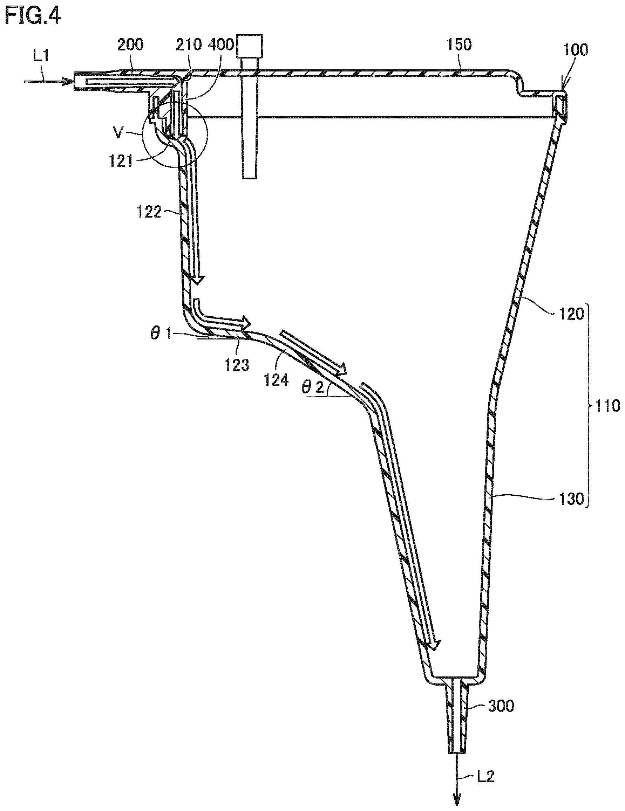

Fig. 1 is a perspective view of a liquid storage reservoir according to an embodiment of the present invention. Fig. 2 is a plan view of the liquid storage reservoir shown in Fig. 1. Fig. 3 is a rear view of the liquid storage reservoir shown in Fig. 1. Fig. 4 is a cross sectional view along a line IV-IV in Figs. 2 and 3. This liquid storage reservoir 10 is used for an artificial heart-lung circuit in an artificial heart-lung machine. Liquid storage reservoir 10 is particularly preferably used as a reservoir for a cardiac muscle protection liquid.

As shown in Figs. 1 to 4, liquid storage reservoir 10 of the present embodiment includes a housing 100, an inflow port 200, an outflow port 300, and an introduction pipe 400.

Housing 100 can store a liquid (such as a cardiac muscle protection liquid). Housing 100 is provided with an attachment portion 20 for attaching an artificial lung, which is a part of the artificial heart-lung machine. Housing 100 has a main body 110 and a cover portion 150.

Main body 110 opens upward. Main body 110 is composed of, for example, a resin. Main body 110 has a trunk portion 120 and a tubular portion 130.

Trunk portion 120 is formed to have a shape of substantially quadrangular tube. Details of trunk portion 120 will be described later.

Tubular portion 130 is connected to a lower end portion of trunk portion 120. Tubular portion 130 has a shape with a cross section gradually decreased in a downward direction.

Cover portion 150 is connected to an upper end portion of main body 110, and closes the opening of main body 110. Cover portion 150 is composed of, for example, a resin. A port to be used for supply of a liquid medicine or the like is connected to cover portion 150.

Inflow port 200 is connected to an upper portion of housing 100, more specifically, to cover portion 150. Inflow port 200 is a port to allow the liquid to flow into housing 10. A circulation inflow line L1 is connected to inflow port 200.

Inflow port 200 is connected to housing 100 in a posture intersecting the vertical direction. In the present embodiment, inflow port 200 is connected to cover portion 150 in a posture (horizontal posture) orthogonal to the vertical direction. As shown in Fig. 4, inflow port 200 has a shape with an inner diameter gradually decreased in a direction toward a downstream-side end portion of inflow port 200. However, the inner diameter of inflow port 200 may be unchanged in a long-side direction thereof. As shown in Fig. 4, the downstream-side end portion of inflow port 200 has a wall surface 210 opposed to flow of the liquid. Wall surface 210 is preferably provided in a direction vertical to the flow of the liquid, but is not limited thereto.

Outflow port 300 is connected to a lower portion of housing 100, more specifically, to a lower end portion of tubular portion 130. Outflow port 300 is a port to allow the liquid to flow out of housing 100. A circulation outflow line L2 is connected to outflow port 300. The liquid having flowed out of outflow port 300 flows into housing 100 from inflow port 200 via circulation outflow line L2 and circulation inflow line L1.

Introduction pipe 400 is connected to the downstream side of inflow port 200, more specifically, to the downstream-side end portion of inflow port 200 so as to introduce the liquid into housing 100. Introduction pipe 400 has a shape extending vertically downward from the downstream-side end portion of inflow port 200. Introduction pipe 400 is formed to be tubular. Introduction pipe 400 has a shape with an inner diameter gradually increased in the downward direction. However, the inner diameter of introduction pipe 400 may be unchanged in a long-side direction thereof.

Here, trunk portion 120 will be described. As shown in Fig. 4, trunk portion 120 has an opposed wall 121, a guide wall 122, an intersecting wall 123, and a coupling wall 124.

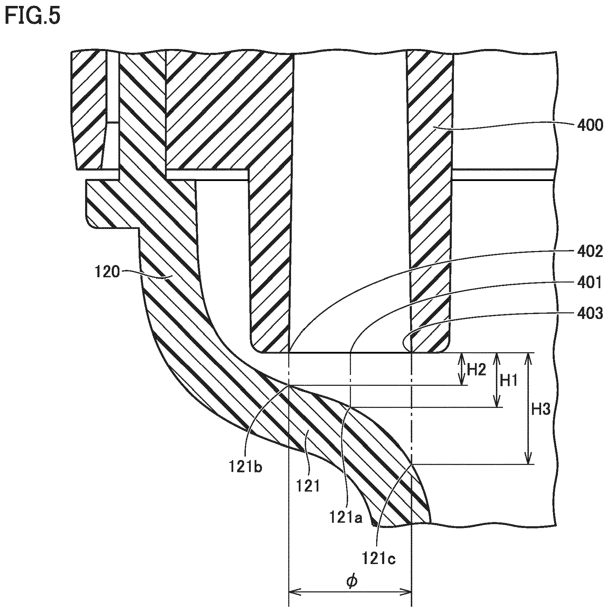

Opposed wall 121 is formed at a position opposed to the lower end portion of introduction pipe 400. As shown in Fig. 5, opposed wall 121 has a shape that extends gradually downward in a direction toward the inner side of trunk portion 120 (in a direction toward guide wall 122). In the present embodiment, opposed wall 121 has a shape curved to protrude obliquely upward (right upward in Fig. 5). More specifically, when viewed in a cross sectional view, opposed wall 121 is curved to have an inflection point at which the inclination of opposed wall 121 is changed from a positive value to a negative value at the lower end portion of introduction pipe 400.

Guide wall 122 guides the liquid downward from opposed wall 121. Guide wall 122 is formed to have a shape of flat plate.

Intersecting wall 123 has a shape extending from the lower end portion of guide wall 122 in a direction intersecting guide wall 122. Intersecting wall 123 has a shape slightly inclined to gradually extend downward in a direction away from guide wall 122.

Coupling wall 124 couples intersecting wall 123 and tubular portion 130 to each other. Coupling wall 124 has a shape inclined to extend gradually downward in a direction toward tubular portion 130. As shown in Fig. 4, an angle θ2 between coupling wall 124 and the horizontal direction is larger than an angle θ1 between intersecting wall 123 and the horizontal direction.

As shown in Fig. 5, opposed wall 121 has a center-opposed portion 121a located vertically below a center 401 of the lower end portion of introduction pipe 400. A distance H1 between center 401 of the lower end portion of introduction pipe 400 and center-opposed portion 121a is preferably 0.1 time or more and 0.8 time or less, more preferably 0.4 time or more and 0.6 time or less, as large as an inner diameter ϕ of the lower end portion of introduction pipe 400. In the present embodiment, distance H1 is set to 0.42 time as large as inner diameter ϕ of the lower end portion of introduction pipe 400. An angle between the central axis of introduction pipe 400 and a line tangent to center-opposed portion 121a is preferably set to about 30° to 75°.

A distance H2 between an upper end portion 121b of opposed wall 121 and a portion 402 of the lower end portion of introduction pipe 400 opposed to upper end portion 121b is preferably 0.05 time or more and 0.4 time or less, more preferably 0.2 time or more and 0.3 time or less, as large as inner diameter ϕ of the lower end portion of introduction pipe 400. In the present embodiment, distance H2 is set to 0.26 time as large as inner diameter ϕ of the lower end portion of introduction pipe 400. An angle between the horizontal direction and a line tangent to upper end portion 121b is smaller than an angle between the horizontal direction and the line tangent to center-opposed portion 121a.

A distance H3 between a lower end portion 121c of opposed wall 121 and a portion 403 of the lower end portion of introduction pipe 400 opposed to lower end portion 121c is preferably 0.2 time or more and 1.6 times or less, more preferably 0.8 time or more and 1.2 times or less, as large as inner diameter ϕ of the lower end portion of introduction pipe 400. In the present embodiment, distance H3 is set to 0.89 time as large as inner diameter ϕ of the lower end portion of introduction pipe 400. An angle between the horizontal direction and a line tangent to lower end portion 121c is larger than the angle between the horizontal direction and the line tangent to center-opposed portion 121a.

Preferably, distance H1, distance H2, and distance H3 satisfy H2 < H1 < H3. Distance H1 is preferably 1.1 times or more and 3 times or less as large as distance H2, and distance H3 is preferably 1.1 times or more and 5 times or less as large as distance H2.

In the above-described liquid storage reservoir 10 of the present embodiment, the liquid having flowed from circulation inflow line L1 into inflow port 200 is hit against wall surface 210 of inflow port 200. Therefore, the flow velocity of the liquid flowing toward the inner side of introduction pipe 400 is decreased. Thereafter, the liquid flows in introduction pipe 400 and is hit against opposed wall 121 formed below introduction pipe 400. Therefore, the flow velocity of the liquid flowing toward the inner side of housing 100 is further decreased. Further, since the liquid having been hit against opposed wall 121 flows downward along guide wall 122, intersecting wall 123, and coupling wall 124 as indicated by arrows in Fig. 4, bubbles are suppressed from being formed in the liquid flowing out of outflow port 300.

### [Implementations]

It will be appreciated by those skilled in the art that the illustrative embodiment described above is a specific example of the following implementations.

A liquid storage reservoir according to an aspect of the present disclosure includes: a housing that is able to store a liquid; an inflow port connected to an upper portion of the housing to allow the liquid to flow into the housing; an outflow port connected to a lower portion of the housing to allow the liquid to flow out of the housing; and an introduction pipe connected to a downstream-side end portion of the inflow port to introduce the liquid into the housing, wherein the inflow port is connected to the housing in a posture intersecting a vertical direction, the introduction pipe is formed to be tubular, and has a shape extending vertically downward from the downstream-side end portion of the inflow port, the housing has an opposed wall formed at a position opposed to a lower end portion of the introduction pipe, and a guide wall that guides the liquid downward from the opposed wall.

In this liquid storage reservoir, since the liquid (such as a cardiac muscle protection liquid) having flowed thereinto from the inflow port is hit against the downstream-side end portion of the inflow port, flows in the introduction pipe, and is then hit against the opposed wall formed below the introduction pipe, the flow velocity of the liquid flowing toward the inner side of the housing is decreased, and the liquid having been hit against the opposed wall flows downward along the induction wall, thereby suppressing formation of bubbles in the liquid flowing out of the outflow port.

Preferably, the downstream-side end portion of the inflow port has a wall surface opposed to flow of the liquid. With this, the liquid having flowed into the inflow port is hit against the wall surface before flowing into the introduction pipe, with the result that the flow velocity of the liquid is more securely decreased before the liquid flows into the introduction pipe.

Further, preferably, the opposed wall has a shape that gradually extends downward in a direction toward the guide wall.

Further, preferably, the opposed wall has a center-opposed portion located vertically below a center of the lower end portion of the introduction pipe, and a distance between the center of the lower end portion of the introduction pipe and the center-opposed portion is 0.1 time or more and 0.8 time or less as large as an inner diameter of the lower end portion of the introduction pipe.

Further, the inflow port may have a shape with an inner diameter gradually decreased in a direction toward the introduction pipe.

Further, the introduction pipe may have a shape with an inner diameter gradually increased in a downward direction.

In this implementation, the flow velocity of the liquid is decreased in the direction toward the downstream side of the introduction pipe, thereby suppressing formation of bubbles more securely.

Preferably, the housing further has an intersecting wall having a shape extending from a lower end portion of the guide wall in a direction intersecting the guide wall.

With this, the liquid flowing downward along the side wall is hit against the intersecting wall to decrease the flow velocity of the liquid, thereby further suppressing formation of bubbles.

Further, preferably, the housing further has a tubular portion provided below the intersecting wall, the outflow port is connected to a lower end portion of the tubular portion, and the tubular portion has a shape having a cross section gradually decreased in a direction toward the outflow port.

With this, the liquid surface of the liquid in the tubular portion is likely to be high, thus resulting in a small distance from the intersecting wall to the liquid surface when the liquid surface is located in the tubular portion below the intersecting wall. Therefore, the formation of bubbles is effectively suppressed when the liquid flowing downward from the intersecting wall reaches the liquid surface in the tubular portion.

In this case, preferably, the housing further has a coupling wall that couples the intersecting wall and the tubular portion to each other, and the coupling wall has a shape inclined to extend gradually downward in a direction toward the tubular portion.

It should be noted that the embodiments disclosed herein are illustrative and non-restrictive in any respect. The scope of the present invention is defined by the terms of the claims, rather than the embodiments described above, and includes any modifications within the scope and meaning equivalent to the terms of the claims.

### REFERENCE SIGNS LIST

10: liquid storage reservoir; 100: housing; 110: main body; 120: trunk portion; 121: opposed wall; 121a: center-opposed portion; 122: guide wall; 123: intersecting wall; 124: coupling wall; 130: tubular portion; 150: cover portion; 200: inflow port; 210: wall surface; 300: outflow port; 400: introduction pipe; L1: circulation inflow line; L2: circulation outflow line.

## Claims

1. A liquid storage reservoir comprising:
a housing that is able to store a liquid;
an inflow port connected to an upper portion of the housing to allow the liquid to flow into the housing;
an outflow port connected to a lower portion of the housing to allow the liquid to flow out of the housing; and
an introduction pipe connected to a downstream-side end portion of the inflow port to introduce the liquid into the housing, wherein
the inflow port is connected to the housing in a posture intersecting a vertical direction,
the introduction pipe is formed to be tubular, and has a shape extending vertically downward from the downstream-side end portion of the inflow port,
the housing has
an opposed wall formed at a position opposed to a lower end portion of the introduction pipe, and
a guide wall that guides the liquid downward from the opposed wall.

2. The liquid storage reservoir according to claim 1, wherein the downstream-side end portion of the inflow port has a wall surface opposed to flow of the liquid.

3. The liquid storage reservoir according to claim 1 or 2, wherein the opposed wall has a shape that gradually extends downward in a direction toward the guide wall.

4. The liquid storage reservoir according to any one of claims 1 to 3, wherein
the opposed wall has a center-opposed portion located vertically below a center of the lower end portion of the introduction pipe, and
a distance between the center of the lower end portion of the introduction pipe and the center-opposed portion is 0.1 time or more and 0.8 time or less as large as an inner diameter of the lower end portion of the introduction pipe.

5. The liquid storage reservoir according to any one of claims 1 to 4, wherein the inflow port has a shape with an inner diameter gradually decreased in a direction toward the introduction pipe.

6. The liquid storage reservoir according to any one of claims 1 to 5, wherein the introduction pipe has a shape with an inner diameter gradually increased in a downward direction.

7. The liquid storage reservoir according to any one of claims 1 to 6, wherein the housing further has an intersecting wall having a shape extending from a lower end portion of the guide wall in a direction intersecting the guide wall.

8. The liquid storage reservoir according to claim 7, wherein
the housing further has a tubular portion provided below the intersecting wall,
the outflow port is connected to a lower end portion of the tubular portion, and
the tubular portion has a shape having a cross section gradually decreased in a direction toward the outflow port.

9. The liquid storage reservoir according to claim 8, wherein
the housing further has a coupling wall that couples the intersecting wall and the tubular portion to each other, and
the coupling wall has a shape inclined to extend gradually downward in a direction toward the tubular portion.
